# EUROPEAN PATENT SPECIFICATION

(11) **EP 2 967 967 B1**
(45) Date of publication and mention of the grant of the patent: **25.09.2019**
(21) Application number: 14763022.2
(22) Date of filing: 15.03.2014
(51) Int. Cl.: A61F 5/44, A61M 25/00, A01P 1/00, A61L 29/16, A61L 29/02, A61L 29/04, A61L 29/06, A61L 29/08, A61M 25/02, A61M 39/02, A61M 25/01

(54) **SYSTEMS AND METHODS FOR MICROBIAL RESISTANCE ZONES**
SYSTEME UND VERFAHREN FÜR MIKROBENBESTÄNDIGKEITSZONEN ZUR SELEKTIVEN BEREITSTELLUNG PROPHYLAKTISCHER UND THERAPEUTISCHER WIRKSTOFFE
SYSTÈMES ET MÉTHODES DESTINÉS À DES ZONES DE RÉSISTANCE MICROBIENNE

(30) Priority: 15.03.2013 US 201361852228 P; 27.11.2013 WO PCT/US2013/072304
(43) Date of publication of application: 20.01.2016
(73) Proprietor: Griffith, Donald, Houston, TX 77056 (US)
(72) Inventor: Griffith, Donald, Houston, TX 77056 (US)
(74) Representative: Mewburn Ellis LLP
(86) International application number: PCT/US2014/029933
(87) International publication number: WO 2014/145211

(56) References cited:
- WO-A1-92/08499
- WO-A1-98/52494
- WO-A2-2011/146699
- WO-A2-2012/131351
- US-A- 4 976 735
- US-A- 5 234 408
- US-A- 5 766 249
- US-A- 5 766 249
- US-A- 5 837 275
- US-A- 5 861 035
- US-A1- 2005 129 732
- US-A1- 2007 299 409
- US-A1- 2010 126 404
- US-B2- 8 105 520
- US-B2- 8 118 736

## Description

### Technical Field

The present disclosure generally relates to microbial resistance systems and methods, and more particularly relates to methods and systems for retarding (and potentially freeing from) infection in connection with temporary and permanently implanted medical devices positioned intracorporeally in humans and animals.

### Background

The present disclosure describes certain embodiments in connection with urinary catheters placed transurethrally or transcutaneously, and other or related catheters and devices placed transcutaneously or via natural body orifices into internal organs and spaces. Infections are commonly associated with such catheters and devices employed in humans and animals. Prevention of these infections is desirable. Catheters and their uses are well known in both the medical and veterinary medical fields. Various types of catheters include urinary catheters, venous catheters, arterial catheters, nephrostomy catheters, dialysis catheters, transcutaneous cardiac powerlines, and others. Such devices typically allow access to the interior of an animal body in order to drain fluids, to deliver fluids, or to provide access for medical instruments to perform a desired function. These devices can provide a wide array of medical benefits to patients. Use of such devices in human health care is evident in light of data that approximately 50% of the patients in nursing homes are placed there because of bladder dysfunction and the need for diapers, perineal pads or chronic tube drainage-i.e. the use of a urinary catheter to drain urine from the bladder. There are more than 20 million urinary catheters sold in the USA, annually.

Urinary catheters are one example of a temporary indwelling catheter, that is, a catheter that is inserted into the patient and remains for a limited period of time (commonly 30 days, for example). Other examples of temporary indwelling catheters include, but are not limited to, arterial and venous catheters used for taking repeated arterial blood samples, delivering therapeutic agents or for directly monitoring blood pressure, oxygenation, or other blood characteristics. Such catheters create a known risk for infection because they provide a pathway for the migration of resident commensal skin bacteria and other microorganisms from the patient's skin to the interior of the body. This risk is illustrated by the finding that virtually 100% of the indwelling urinary catheters become colonized within 60 days of catheter placement. Further, indwelling arterial catheters are used predominantly in controlled environments, such as hospitals or other medical facilities, to prevent colonization of the catheter. These arterial catheters pose a significant risk of transmitting infection to the patient's blood and are closely monitored so that such infection is quickly detected and efforts commenced to treat the infection.

The principal source of such blood stream and device infections is often skin borne bacteria which have continuous access to the catheters that penetrate the skin or pass through normal body orifices. Such bacteria, including yeast and other microorganisms, may attach to the catheter's external surface and immediately reproduce. Within 24 hours of such attachment, high densities of resident skin bacteria have typically attached and replicated on each square centimeter of catheter external surface that contacts skin or adjacent mucosal surfaces.

Once skin microbes have attached and commence replication on catheter external surfaces, mucous from urethra, urine, or other tissue fluids adjacent to the catheter's external surface facilitates, aids and stimulates bacteria migration inward towards and into the bladder lumen or other normally sterile intra-corporeal space. This initial migration typically occurs predominantly on the external catheter surface, though the same may and often does occur on the catheter's lumenal surface. Bacteria may also infect and colonize the interior wall of a urinary catheter. For example, when a urinary collection bag is held above the bladder, colonized bag urine may drain retrograde into the bladder lumen. In any event, catheters and other devices can allow and facilitate migration of microbes leading to infection.

Clinical studies demonstrate that within 30 to 60 days of implantation, virtually all patients with urinary catheter drainage develop urinary infection. Antibiotics delay but do not prevent such infection. Antibiotics reduce colonization rates from approximately 5 percent per day down to two to three percent per day. The route of antibiotic deliverysystemic (i.e. oral or intravenous), topical as catheter coating, or topical as injection into the bladder lumen -- slows and delays but does not prevent colonization of intravesical urine. Thus, virtually all patients on continuous antibiotics develop urinary infection within 30 to 60 days of catheter placement.

With respect to urinary catheters, the infections appear to occur regardless of whether the catheter is placed in the urethra or as a suprapubic abdominal catheter. Other transcutaneous and trans-natural orifice catheters become similarly colonized in use. This phenomenon of universal colonization of transcutaneous and trans-natural orifice devices is largely a consequence of "open external surface access" from skin to internal organ or cavity. Tenkhoff peritoneal dialysis catheters and some chronic arterial catheters have attempted "closed external surface access" by placing permanently attached porous polyester (e.g., Dacron®) cuffs on segments of catheter passing through body wall musculature. Tenkhoff catheters employed in peritoneal dialysis and using "closed external surface access" have appeared to colonize with microbes less frequently than "open external surface access" devices. Certain efforts show that, in the Tenkhoff "closed external surface access" systems, colonization occurs at a frequency of approximately 1% in 12 months, whereas urinary catheters typically become colonized at a much greater frequency (e.g., virtually 100%) in 1-2 months.

One possible reason that prophylactic antibiotic treatments are unsuccessful is that bacteria sensitive to the employed antibiotic are killed, however antibiotic resistant bacteria and non-bacterial microorganisms, such as yeast, remain on the skin and thereby on the adjacent and open access urinary catheters. In such circumstances, additional treatment, such as treatment by a second antibiotic, an anti-fungal agent, a quorum signaling inhibitor agent, a biofilm dispersive agent, or some combination of such agents, is needed to treat the surviving and colonizing microbes. Some bacteria, such as Proteus species, among others, interact chemically with urinary waste. For example, the enzyme urease from Proteus and some other bacterial species interacts with urinary waste (i.e., urea) to alkalinize urine and increase supersaturation of calcium and magnesium salts. These compounds are normally dissolved in acidic urine, however, they can become problematic if the chemical reaction of enzyme with urine adversely accelerates calcium crystal nucleation and encrustation of urinary catheters and stone formation within the urinary organs.

Most medical device surfaces are fabricated with little or no control of the device surface; such surfaces are relatively smooth. It has been determined in accordance with the present disclosure that micro and nanoscale controlled modulation of selected polymeric, metallic and other material surfaces. For example, controlled Shark-skin like micro-modulated surfaces are in development by Sharklet, Inc. and controlled nano-pillared surfaces are in development by SiOnyx, Inc. Selected polymer and metallic surfaces can be fabricated with such "controllably textured surfaces." Microbial attachment or migration is often the result of smooth surfaces of conventional devices. Improved surface structures, patterns or other modulations may discourage, rather than encourage, microbial attachment or migration. It would be also be an improvement to provide surfaces of devices that can kill selected microbial species. It has been determined, in accordance with the present disclosure, that improved controlled fabrication of modulated [i.e. textured] surfaces may provide resistance to microbes, such as in zones or regions of devices, and may allow addition of elutable and non-elutable coatings and other antimicrobial improvements.

Bacteria may exist and grow as individual cells floating in a liquid environment or as a colony adherent to a surface. In these two different forms, floating and adherent, the metabolism of the bacteria can differ according to the form. Such differences are caused, at least in part, by quorum signaling that results from the cell to cell interactions of individual bacteria in the colony as well as by the higher concentration of waste products. The ultimate result of colony growth, quorum signaling, and other factors is that bacterial cells, microbial waste, and dead patient tissue accumulate on the catheter surfaces in a metabolically active, slick, gelatinous biofilm. These biofilms are sanctuaries within which the bacteria may be one thousand to ten thousand times more resistant to antibiotics than in the case of individually floating and growing bacterial cells. Through continued growth, the dense microbial-packed biofilms migrate slowly on the catheter surfaces toward the bladder lumen. The result is substantially 100% colonization of urine in patients with chronic tubes longer than 30 to 60 days.

Once a biofilm is established on the catheter surface, the infection cannot be readily eradicated from the indwelling catheter. Rather, the patient must be treated with antibiotics and the biofilm infected catheter must be removed and replaced.

Attempts have been made to reduce or eliminate the incidence of catheter-associated infections. Such efforts typically involve the use of chemical agents to treat the catheter entry site or the catheter body in order to kill the invading microorganism. However, such efforts have been unsatisfactory, particularly for catheters that remain in place after the patient leaves the medical facility.

It would therefore be a significant improvement in the art and technology to provide systems and methods for reducing and preventing the formation and the migration of biofilms on surfaces of medical or veterinary devices, such as catheters. Further, it would be a significant improvement to provide treatment of such surfaces with multiple agents effective to kill infecting bacteria, prevent or limit the spread of infecting bacteria, and prevent or disrupt the formation of biofilms thereon. Still further, it would be a significant improvement to provide an improved tissue bonding cystostomy device in which the catheter is protected from skin infection by a barrier that may be removed without removing the catheter from the bladder lumen.

### Summary

Disclosed is a cystostomy device for implant in a patient. The device includes a transcutaneous sleeve with an outer surface and an inner surface, a flange connected to the transcutaneous sleeve, adapted to reduce contamination of the outer surface of said transcutaneous sleeve, a catheter defining a passage for the flow of fluids from within the patient to outside the patient, the inner surface of the transcutaneous sleeve defining a path through which the catheter passes, and an anchor adapted for implantation into the biological tissue of the patient, the anchor selectively connecting to the catheter and the transcutaneous sleeve.

Disclosed is a cystostomy device. The device includes a transcutaneous sleeve, with an outer surface and an inner surface, the outer surface lying adjacent to skin, fat, muscle or other biological tissue, and the inner surface defining a path, at least one microbe resistance zone of the transcutaneous sleeve, a flange adapted to reduce contamination of the outer surface of said transcutaneous sleeve, a catheter defining a passage for the flow of fluids from the bladder to a surface of the patient's skins, the path of the inner surface of the transcutaneous sleeve allows passage of catheter, and an anchor adapted for implantation into biological tissue. The transcutaneous sleeve is connected to the anchor and the flange and the drainage tube is removably connectable to the anchor.

Disclosed is a catheter. The catheter includes a first end extending to an outer surface of a patient, a transcutaneous section connected to the first end, a second end extending into the bladder lumen of a patient, and at least one microbe resistance zone selected from the group of: a microbe resistance zone of the first end, a microbe resistance zone of the transcutaneous section, a microbe resistance zone of the second end, and combinations.

Disclosed is a urinary catheter. The catheter includes a first end extending from an exterior surface of a patient, a second end extending into the bladder lumen of said patient, and a transcutaneous section connecting said first end to said second end. The transcutaneous section further includes an interior surface defining a pathway for the flow of fluids from the bladder lumen to the exterior of said patient, an exterior surface, and a plurality of microbe resistance zones comprising a first microbe resistance zone containing an anti-biofilm surface modulation and a second microbe resistance zone selected from the group consisting of anti-biofilm surface modulations, non-elutable molecular agents, nanochannel delivered molecular agents, and elutable molecular agents.

Yet another embodiment of the invention is a medical device including a microbe resistance zone selected from the group of: surface modulations, antibiotic, antifungal, antiseptic, biofilm inhibitor, dispersing agent, a detector, an enzyme-linked immunosorbent assay, elutable coating, non-elutable coating, and combinations.

Disclosed is a tape including a substrate and an antimicrobial eradicator laminated atop the substrate.

### Brief Description Of The Drawings

The present invention is illustrated by way of example and not limitation in the accompanying figures, in which like references indicate similar elements, and in which:
Fig. 1 illustrates an improved tissue bonding cystostomy device, according to certain embodiments of the invention;
Fig. 2 illustrates an improved Tissue Bonding Cystostomy device placed in a patient, according to certain embodiments of the invention;
Fig. 3a illustrates a side view of a transcutaneous sleeve, according to certain embodiments of the invention;
Fig. 3b illustrates an end view of a transcutaneous sleeve, according to certain embodiments of the invention;
Fig. 3c illustrates a top view of a flange of a transcutaneous sleeve, according to certain embodiments of the invention;
Fig. 4a illustrates a cross-section taken along a diameter of a drainage catheter having a series of nano-channels and antimicrobial eradication therapies formed in an outer surface, according to certain embodiments of the invention;
Fig. 4b illustrates a cross-section taken along a length of a drainage catheter having a series of nano-channels and antimicrobial eradication therapies formed in an outer surface, according to certain embodiments of the invention;
Fig. 5 illustrates an external side view of a drainage catheter, according to certain embodiments of the invention;
Fig. 6 illustrates a urethral (or "Foley") catheter with microbe resistance zones, according to certain embodiments of the invention;
Fig. 7 illustrates certain exemplary nano-scale surface structures for devices, according to certain embodiments of the invention;
Fig. 8 illustrates a perspective view of a tape roll for applying a microbial resistance zone (MRZ) to a device, according to certain embodiments of the invention; and
Fig. 9 illustrates a partial cross sectional view of an exemplary tape for applying a MRZ to a device, according to certain embodiments of the invention.

### Detailed Description

The following is a detailed description of example embodiments of the invention depicted in the accompanying drawings. The amount of detail offered is not intended to limit the anticipated variations of embodiments; on the contrary, the intention is to cover all modifications, equivalents, and alternatives falling within the scope of the present disclosure as defined by the appended claims.

### Tissue Bonding Cystostomy Device and Sleeve

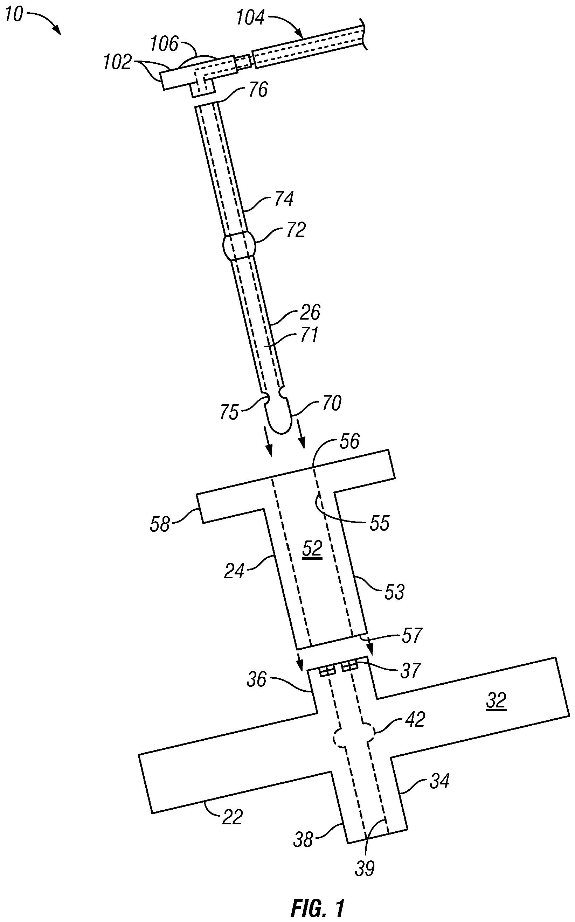

Referring initially to Fig. 1, an isometric view of an embodiment of a Tissue Bonding Cystostomy (TBC) device 10, usable within the scope of the present disclosure, is shown. The depicted TBC 10 includes an anchor 22, a transcutaneous sleeve 24 and a catheter 26, each of which is described in greater detail below.

In one embodiment, the anchor 22 may comprise an anchor implant with a porous surface usable to hold the catheter 26 in place relative to the bladder and the bladder lumen. For example, the anchor 22 may be sandwiched between rectus muscle 62 and bladder muscle 64 and secured in place. Fibroblasts from rectus muscle 62 and bladder muscle 64 can then grow into and bond, robustly and water-tightly, with the pores of the anchor 22, fixing the anchor 22 relative to the two muscles 62, 64.

In another embodiment, the anchor 22 is comprised of non-porous material, such as a metal or a polymer polyurethane, polytetrafluroethylene (PTFE), silicon, or other materials acceptable for medical and/or veterinary applications. In such embodiments, the non-porous material may be coated with a highly porous PTFE, polyester, metal or another polymeric and/or similarly porous material.

The anchor 22 is shown having a disc-shaped base 32 and a tubular section 34 extending from the approximate center of the disk-shaped base 32. The tubular section 34 may be integral to the disc-shaped base 32 or may be one or more parts extending from and/or through the disc-shaped base 32. Both sides of the disk-shaped base 32 and the lower portion 38 of the tubular section 34 adjacent to the disk-shaped base 32 can be coated with the highly porous PTFE, polyester or similar porous material. In certain embodiments, the upper portion 36 of the tubular section 34 is not comprised of or coated with porous PTFE or similar material, and is therefore not subject to bonding with adjacent muscle tissue.

The tubular section 34 may include a locking mechanism 42, on the inside diameter 39 in some embodiments, that cooperates with the catheter 26 to secure the catheter 26 to the anchor 22. The catheter may contain a balloon, tabs, or any other suitable structure to cooperate with the locking mechanism 42 to secure the catheter 26 to the anchor 22 as desired. It will be apparent that by securing the catheter 26 to the anchor 22 and by securing the anchor 22 to the rectus muscle 62 and bladder muscle 64, the catheter will be locked in place relative to the rectus muscle 62 and bladder muscle 64 and, by extension, to the bladder lumen 80.

While the depicted anchor includes separate disc-shaped base 32 and upper portion 36 and lower portion 38 of the tubular section 34, embodiments usable within the scope of the present disclosure can include a base of virtually any shape that is operable to securely position the anchor 22 to a desired location within the patient. Further, the anchor 22 may comprise a base in which the tubular section 34 penetrates the base in a location apart from the center of the base or does not penetrate the base at all.

The transcutaneous sleeve 24 includes an inner passage 52, an outer wall 53, an inner wall 55, an upper end 56 and a lower end 57. The inner passage 52 extends along the length of the transcutaneous sleeve 24 for permitting passage of a catheter 26 and therefore may have a diameter at least as large as the outer diameter of the catheter 26.

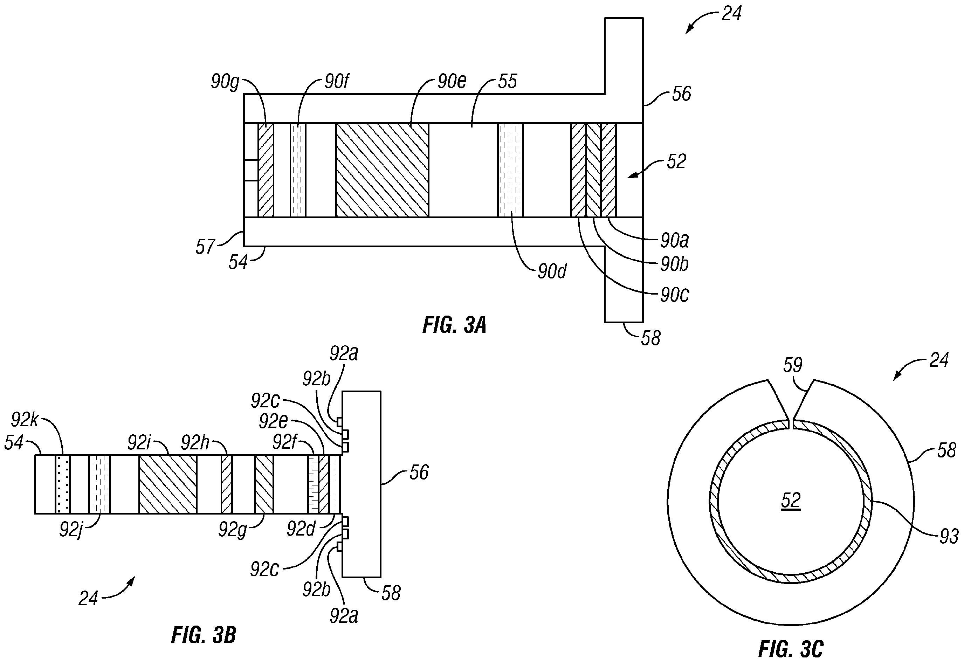

The upper end 56 may have a flange 58 which is placed in contact with the skin surface when the transcutaneous sleeve 24 is placed in the patient. The transcutaneous sleeve 24 is therefore cut such that the distance from the lower end 57 to the upper end 56 allows the sleeve 24 to extend from skin through the subcutaneous fat and dermis to the tip of the tubular section 34 of the anchor 22. In an embodiment, the flange 58 of the transcutaneous sleeve 24 can have a V-shaped notch 59, as shown in Figure 3c. The notch assists in crimping of the transcutaneous sleeve 24, thereby causing the transcutaneous sleeve 24 to be more easily inserted through an incision in the patient. The sleeve 24 may be employed, as later described, to provide microbial resistance zones (MRZs) to subcutaneous fat, which is particularly susceptible to microbial colonization. The TBC 10 may also be used without the transcutaneous sleeve 24. The decision whether to use the sleeve 24 is, for example, an elective option of the attending physicians or surgeon.

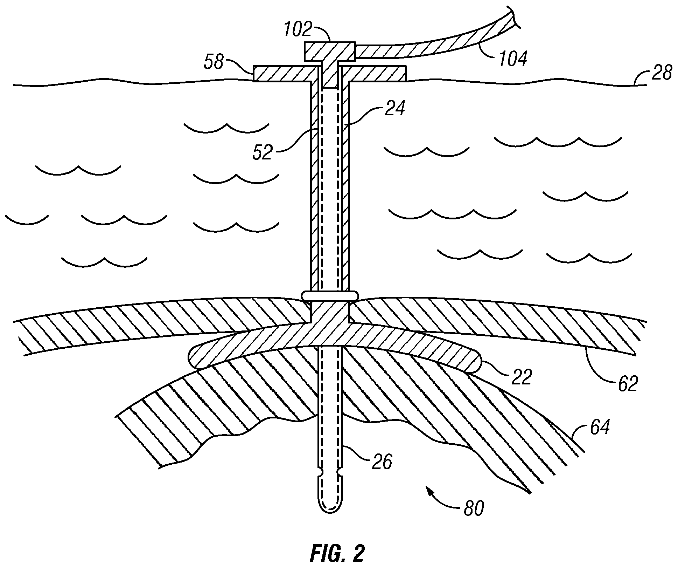

The catheter 26 of the embodiment shown in Figure 1 creates a watertight, microbial-migration-preventive drainage system when installed as a part of the TBC 10. The catheter 26 comprises a body 70 with an external surface 74, a flow path 71 for the flow of bodily fluids, such as urine from the bladder to the skin as well as an inlet 75 providing fluid communication between the flow path 71 and external surface 74 of the catheter 26. It will be appreciated that, in a urinary catheter, the inlet 75 will typically reside in the bladder lumen 80 FIG 2 to allow urine to enter the flowpath 71. The flowpath 71 may have a diameter sufficient to house wires or electrodes, thereby providing for insertion into the bladder and use of electrical instruments. The catheter 26 may have a protrusion 72, such as a bulb, ring, tabs, or the like, extending from the external surface 74. As the catheter 26 is inserted through the transcutaneous sleeve 24 into the tubular section 34 of the anchor 22, the protrusion 72 may contact the locking mechanism 42 of anchor 22, securing the catheter 26 in place. In some embodiments, engagement of the locking mechanism by the protrusion 72 creates a water tight seal between the catheter 26 and the anchor 22. The lower segment of anchor 34 may be tapered so as to automatically stop catheter insertion into the anchor so as to align the locking mechanism 72 of the drainage tube 26 with the locking mechanism of the anchor 42.

Catheters in the embodiments include, without limitation, the use of a protrusion that is bulb shaped, an inflatable protrusion such as a balloon, or any device or feature suitable for use with an anchor 22 to lock the catheter 26 into place. Alternatively, the catheter 26 and/or anchor 22 may contain sealing elements separate from protrusions that cooperate with the locking mechanism 42. In yet further embodiments, the catheter 26 may pass through the anchor 22 in a manner that is not strictly water tight. In some embodiments, the locking mechanism 42 may engage a protrusion 72 in a reversible manner, allowing the catheter 26 to be disconnected from the anchor 22 and removed.

The length of the catheter 26 below the location of the protrusion 72 may be selected such that it is of sufficient length to extend into the patients' bladder to a desired depth. In an embodiment, the catheter 26 can be formed from polyurethane, PTFE, silicone, other similar materials, or combinations thereof.

The TBC 10 may also be used in conjunction with external drainage components 100. For example, external draining components such as a cap 102 and drainage tube 104 may be insertable into the upper end 76 of the catheter 26. In one embodiment, the external drainage components 100 are insertable into the transcutaneous sleeve 24 as well as the catheter 26. In some embodiments, insertion of the external drainage components 100 creates a water tight seal with the catheter 26, the transcutaneous sleeve 24, or both. The cap 102 and drainage tube 104 may include one or more antimicrobial filters to prevent bacteria from entering the inner passage 52 of the transcutaneous sleeve 24 and/or the flow path 71 of the catheter 26. The cap 102 and drainage tube 104 can be retained in the catheter using a friction fit or by other methods of mechanical fixation. The cap may also comprise a self-sealing injection port 106 allowing injection of desired fluids or drugs through the catheter 26 and into the bladder lumen 80 (shown in Fig. 2). The self-sealing occlusive cap 102 on the drainage tube 104 also allows use of needle attached to the drainage tube 104, such as for draining urine from the bladder lumen on continuous or intermittent intervals as desired.

With reference to Fig. 2, it will be appreciated that, in the illustrated embodiments, the transcutaneous sleeve 24 isolates the catheter 26 from contact with the skin. Thus the transcutaneous sleeve 24 forms a physical barrier reducing the risk of colonization on the catheter 26. Preventing such colonization of the catheter 26 helps prevent urinary tract infection because only the catheter 26 provides the link from the skin 28 to the bladder lumen 80. Further, the inner passage 52 of the transcutaneous sleeve 24 can be kept clean and dry by proper application and maintenance of the cap 102. Such a clean and dry environment is not favorable for bacterial growth, for biofilm formation or for bacterial migration, and therefore further reduces the likelihood of bladder infection via the catheter 26. In certain embodiments, the cap is large enough to cover the end of inner passage 52 at the upper end 56.

It will also be appreciated that the transcutaneous sleeve 24 is the most susceptible portion of the TBC 10 to infection by microbes on the skin. Therefore, in some embodiments, the transcutaneous sleeve 24 may be replaced without removing the catheter 26 from the patient. To do so, cap 102 and external drainage tube 104 are removed from the catheter 26 and the transcutaneous sleeve 24. The transcutaneous sleeve 24 is then removed from around the catheter 26 with the catheter 26 in place. A new transcutaneous sleeve 24 may then be installed around the catheter 26.

### Microbial Resistance Zones

Another embodiment of the transcutaneous sleeve 24 is illustrated in Figs. 3a thru 3c. The transcutaneous sleeve of Figs. 3a thru 3c has a series of microbial resistance zones (MRZs) in the inner passage 90a through 90g, placed on the outer surface 92d through 92k and on the flange 92a through 92c of the transcutaneous sleeve 24. For purposes of the embodiments, microbial resistance zones, or MRZs, are segments or portions of antimicrobial agent(s) and/or surface structure(s) of devices, which for example include, but are not limited to, antibiotics, biofilm inhibitors, dispersing agents, bacterial or fungal detection agents, or other antimicrobial agents or antibiotic eradication therapies (AETs), and device surface patterns or modulations, such as micro or nano channels, dips, pits, pillars, pores, or humps, bumps, mounds, or protrusions, or Sharklet™ or other textures, or materials, such as black silicon or other nanomaterials. These MRZs retard and potentially free from infection otherwise caused by microbial colonization and migration on devices.

Molecules or agents may be presented as MRZs at the surface of medical devices, including AETs and the like. For example, a Poly(methylacrylic acid), Poly(allylamine hydrochloride), glutaraldehyde (PAA/PAH coating) technique developed by the Stevens Institute of Technology forms a non-elutable coating to which various molecules may be cross-linked and permanently attached to a silicone surface of a device. Additionally, passively elutable coatings such as those described in Palukhina and Sukhisvili, Polymer assemblies for controlled delivery of bioactive molecules and surfaces, Advanced Drug Delivery Reviews v. 63 (9), pp. 822-36 (2011), and others, may form MRZs.

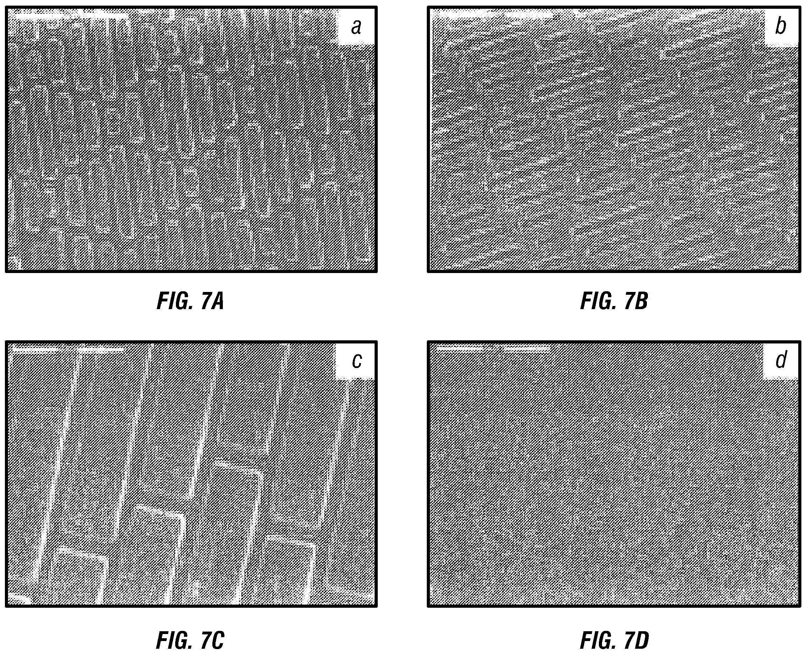

Referring to Fig. 7, the surface of medical devices can further be configured and textured with particular structures as MRZs to retard or influence microbe migration. Surfaces of catheters or other devices can be formed with modulations, such as, for example, micro or nano-scale elevated mounds or pits, for example, 1-2 microns tall or 1-2 microns deep. These elevations and depressions of the surface slow microbial migration. Surfaces can be altered generally horizontally and/or generally vertically (in relation to in situ position in use). For example, vertically, nano-scale pillars that are a fraction of the size of most pathogenic bacteria (e.g., on the order of about 0.25 to about 2 microns long and 1 micron wide) are possible. On the order of 1000 nano-scale pillars can be formed in 1 micron of surface area. Nano-pillars approximately 50-100 nanometers tall spaced in forest-like array less than 200 nanometers apart on the surface of a medical device can puncture the external membrane of microbes as they contact the surface. The microbes are thereby drained of vital nutrients, and once so stabbed, tend to die quickly (e.g., within seconds).

Nano-channels and/or nano-pores, or lines or arrays of nano-channels and/or nano-pores, on the surface of devices can effectively provide time release of molecules from within chambers or pores in or on surfaces of the device. The chambers or pores are merely one example for an MRZ, and other placement of therapy molecules at a specific location on a catheter or on a specific surface of a TBC are included in embodiments. Thus, MRZs provide multiple means by which different molecules can be presented at discrete locations along a component of the TBC, along a Foley or other urinary catheter, or other types of catheter or device.

Fig. 7 illustrates four examples of nano-scale structures/patterns that may be possible for MRZs, however, a wide variety of other shapes and patterns are possible.

In certain embodiments of the sleeve 24, for example, an MRZ 90a, 92d is adjacent to or abuts with the flange 58 in the inner passage 54 or on the outer wall 53 of the transcutaneous sleeve 24 and comprises a Sharklet™ or other surface modulation designed to prevent formation of a biofilm at that location. In another embodiment, an MRZ adjacent the flange 92a, 92d comprises a biofilm inhibitor or dispersing agent such as DispersinB® or deoxyribonuclease I or other effective biomolecules such as lysozyme or others. Placement of such antibiotic eradication therapies (AETs) of the MRZ adjacent to the flange 58 are effective to prevent or greatly reduce colonization of the transcutaneous sleeve 24 because such colonization occurs almost exclusively due to microbial migration with biofilm from skin surface to the intra-corporeal organ or cavity.

Any number of the MRZs (any of one or more) may be formed or included in the surface of devices. In certain embodiments, multiple MRZs may be formed as a "Vertical Gauntlet", with any number of the MRZs serving to deliver more than one AET(s), for example, the AETs are stacked on top of each other on the surface. For example, a surface modulation, such as Sharklet™ surface technology available from Sharklet Technologies, Inc., may be formed or presented in the base or bottom layer of or adjacent the device. A non-elutable coating may then be applied on top of the modulated surface. One or more elutable coatings may further be stacked on top of the non-eluting surface coating at the MRZ.

Coatings of device surfaces may, themselves, be therapeutic, and/or these may bond therapeutically active guest molecules, such as antibiotics gentamicin, biofilm dispersive molecules, quorum signaling inhibitor molecules and /or other AETs. Two examples of coatings that bond active therapeutic agents are Montmorillonite clay platelets and Polypyrillodone. Both are hygroscopic and swell considerably when exposed to water. Montmorillonite can form a non-eluting electrostatic bond with underlying charged polymer surfaces and also bonds to selected guest therapeutic molecules in a non-eluting electrostatic manner. Polypyrillodone is a sticky hygroscopic molecule that adheres to polymer surfaces and swells and absorbs solutes dissolved in solvent. Polypyrillodone and its dissolved guest molecules elute from polymer surfaces over days or weeks, depending upon the volume and rate of surface fluid flow.

Other coatings, and methods of placing the coatings, may also be possible. For example, a PAA/PAH coating may be applied by applying the coatings using an appropriate size of brush. By selecting the size of brush and the location of application, a discreet portion of the catheter or TBC presents the desired agent of the MRZ.

In addition, coating and surface agents and layered agents presented are not limited to antibiotics or antiseptics, such as nitrofurazone, gentamycin or triclosan. Any molecule with a desired effect may be presented at a desired surface location as MRZs. For example, molecules such as dispersinB®, deoxyribonuclease 1, and lysozyme, and others have been identified to inhibit the formation, stability, and/or growth of biofilms on infected surfaces. Such molecules may be useful as MRZs in preventing or limiting catheter associated infections or symptoms associated with such infections.

In addition to molecule presentation in MRZs on surfaces of devices, surface features, such as Sharklet™ or other, can inhibit the formation of biofilms on surfaces. A Sharklet™ and Black Silicone™ surfaces, for example, are textured patterns. Sharklet is modeled after shark skin and shown to reduce the rate at which bacterial biofilm form and migrate on the polymer surface. Although the Sharklet™ surface is an example of possible embodiment for an MRZ, other surface features or surface modulations are also possible. Black Silicone™ pillared surfaces are capable of 'mechanically killing' microbes, by puncture or disruption of the molecule.

In certain embodiments, a series of respective microbial resistance zones, or MRZs, is included in a device surface, and each distinct MRZ includes one or more antimicrobial enhancing technologies or AETs. The AETs which may comprise an MRZ include for example, antiseptics, antibiotics, antifungals, surface modulations, biofilm inhibiting agents, biofilm dispersive agents, bacterial or fungal detection agents. These agents are positional on the exterior surface or the interior surface as MRZs of a catheter, for example, to prevent or limit formation of biofilms. Further, the MRZs may contain different antibiotics such that bacteria resistant to one antibiotic may be susceptible to the additional antibiotic(s).

One or more MRZs may contain multiple AETs. As disclosed above, such AETs may be stacked in sequential layers on the device, tape, or sheet, or, where compatible, may be attached to the same layer. For example, dispersin B and gentamycin may be contained as guest molecules on one layer of elutable or non-elutable coatings in a single MRZ.

In further aspects, one or more MRZs may be configured as a detection mechanism for the presence of bacteria and/or biofilms. For example, bacterial urease releases ammonia when breaking down urea in urine. Therefore, one or more MRZs may contain an alkaline sensitive dye or other color change agent for detection of ammonia. Alternatively, a detection system such as an enzyme-linked immunosorbent assay (ELISA) may be incorporated into the catheter device. According to certain embodiments, one or more MRZs contain permanently bonded antibody fragments which bind to a biofilm molecule or other molecule of microbial origin. A second molecule which binds the same target is placed on the catheter with an elutable coating. The second molecule would preferably contain the active enzyme for the ELISA reaction. However, additional molecules could be provided as additional elutable coatings if desired or required. The reagent for the ELISA, e.g. the substrate for the active enzyme attached to the second molecule, may be releasable, for example, as an additional elutable coating or diffused from one or more nano-tubes or pores, as applicable. Detection of reaction products in such embodiments may, for example, be by simple color change observation at the skin surface or by other means. Other detection systems are envisioned in the scope of certain embodiments.

Referring to Figure 3c, a single MRZ 93 is positioned on the flange 58 of a transcutaneous sleeve 24. Typically, such an MRZ will mirror either the shape of the flange's 58 perimeter, the inner passage 52, or, as here, both, providing a continuous barrier to migration across the flange 58 surface. It will be appreciated, as shown in FIG 3b, that the flange 58 may comprise more than one MRZ.

In another embodiment, the first MRZ 90a may comprise an antibiotic or antiseptic effective to kill at least one, but preferably multiple, bacteria species that migrate from the skin to the surface of the transcutaneous sleeve 24 adjacent to the flange58 In such case, the second MRZ 90b, 92e preferably comprises a surface modulation or a biofilm inhibitor or dispersing agent. In this configuration, the second MRZ prevents or inhibits the migration of colonies which may form from bacteria which are resistant to the treatment presented at the first MRZ 90a or which may evade the first MRZ 90a.

A third or subsequent MRZ 90c through 90f, 92f through 92k may contain one or more components for a detection mechanism such as an enzyme-linked immunosorbent assay (ELISA) or an indicator, such as a dye, that indicates the presence of bacterial waste products such as ammonia, carbon dioxide, lactic acid or other molecules. It will be appreciated that numerous candidates for such indicators exist and any such indicator is useful provided it selectively shows the presence of bacterial growth and is acceptable for use in the patient.

The connection points between the transcutaneous sleeve 24 and the anchor 22 as well as the anchor 22 and the catheter 24 may provide pathways for the bacteria to move into the bladder and other patient tissues. To address this potential, one embodiment of the transcutaneous sleeve 24 may have one or more MRZs 90g, 92k adjacent to the connection point between the anchor 22 and the transcutaneous sleeve 24which comprise at least one nano-channel to deliver antibiotics or antiseptics. A nano-channel delivery system or a micro or nano-channel modulated surface may also be placed adjacent to the point at which the catheter 26 enters a passage through the anchor 22. Such nano-modulated surfaces or nano-channels may be placed on any of the parts of the TBC 10 or other device provided the antibiotic or antiseptic is made available in the desired amounts to the desired location. A benefit of placing micro or nano-modulated surfaces and nano-channels in the transcutaneous sleeve 24 is that this treatment can be resumed by replacing the transcutaneous sleeve 24 without the need for removing the catheter 26.

It will be appreciated that while particular embodiments of the transcutaneous sleeve 24 are described, any device or structure of a device, such as the sleeve or other device, can provide a pathway isolated from the patient's tissue for passage of the catheter 26 from the surface of the skin to the anchor 22. Further, each of the first MRZ 90a, second MRZ 90b and subsequent MRZs may contain any one AET or plurality of AETs and are not limited to the AETs mentioned in the described embodiments.

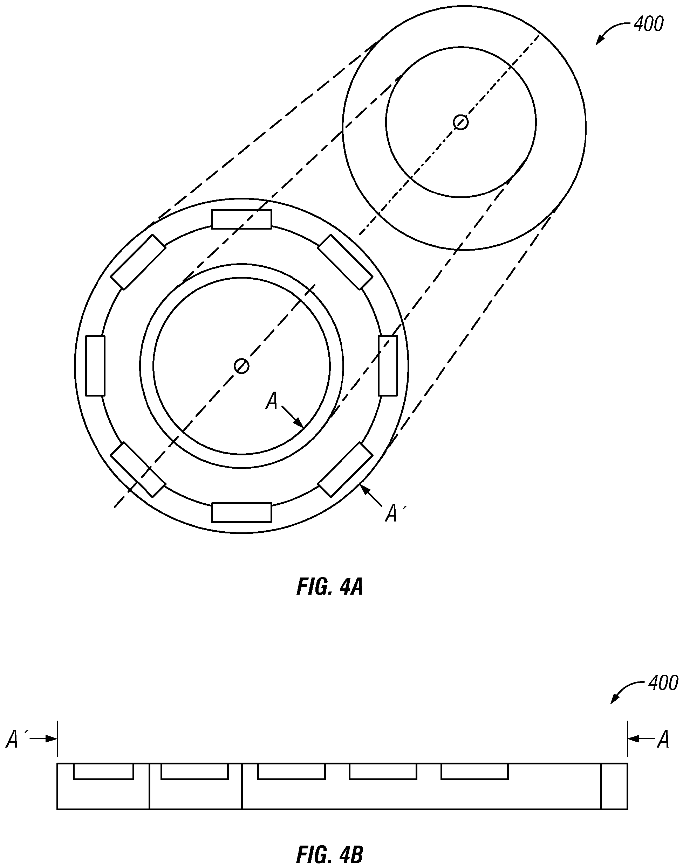

To facilitate the incorporation of an ELISA, other detection mechanism and/or any combination of materials, an MRZ or MRZ's may comprise an array or series of micro or nano-modulated surfaces and/or nano-channels or nano-pores. The nano-channels, nano-pores and chambers which may be in communication thereto can provide continuous production of specific reagents, such as antibody linked enzymes and detection reagents useful in ELISA assays. Further, providing reagents from the nano-channel or nano-pore array may help generate a signal amplification necessary to detect the ELISA reaction product at the skin surface. Reference is made to Figs. 4a and 4b for such an ELISA incorporated in nano-structures of a catheter 400.

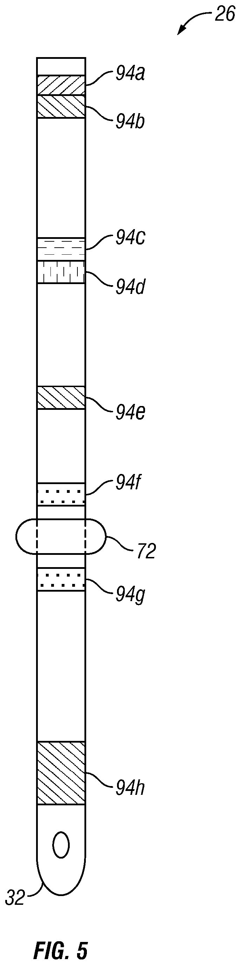

Fig. 5 illustrates a catheter 26. The embodiment shows a series of MRZs 94a through 94h. In the embodiment, one MRZ 94f is located or partially located, adjacent to, but on the skinward side of, the anchor 22 and comprises a surface modulation or a biofilm inhibitor or dispersing agent. In certain embodiments, this MRZ may continue along the catheter 26 as the catheter 26 passes into the anchor 22. The presence of such AETs at this location is designed to prevent or reduce migration of biofilm down the catheter 26 and through the anchor 22. The location of this MRZ 94f must be placed such that it is the proper distance from the tip 32 of the catheter 26, which resides in the bladder lumen. Improper positioning of the MRZ relative to the tip 32 will prevent the MRZ from locating adjacent to the anchor as desired.

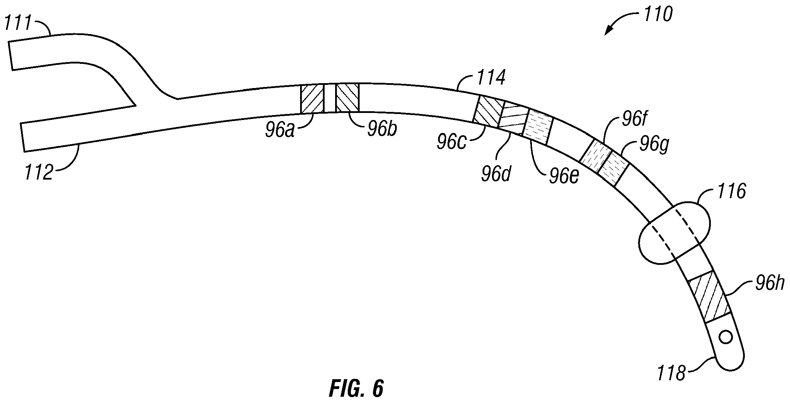

Fig. 6 illustrates a foley type urinary catheter 110 with multiple MRZs. Foley catheters are inserted to follow a patient's urethra in order to reach the bladder lumen. As with the transcutaneous sleeve 24 and cathether 26 of the TBC 10, the embodied catheter 110 of Fig. 6 may comprise one or more MRZs 96a through 96f, with each MRZ having at least one AET. Those familiar with such catheters will appreciate that the MRZs are positioned relative to the tip 118 and balloon 116 of the catheter 110 such that the at least one MRZ 96a is positioned adjacent to the urethral meatus in the male or female patients. Thus, in treating patients, embodiments such as is illustrated in Fig. 5 must be selected with particular attention given to urethral length, such length being affected by gender, age, and other factors. If a catheter 110 of incorrect length is chosen, the MRZs will not be positioned as desired relative to the urethral meatus.

In certain embodiments, the first MRZ 96a of catheter 110 is comprised of at least one of a surface modulation, biofilm inhibitor or dispersing agent in order to prevent or inhibit the attachment, growth, and migration of bacterial colonies on the catheter. The urethral meatus is the entry point for foley catheter associated infections and therefore providing biofilm inhibiting and/or dispersing AETs will assist in preventing bacteria from colonization the catheter at the meatus. In some embodiments, the second MRZ 96b may be an antibiotic or antiseptic to kill any bacteria that pass over or avoid the first MRZ.

The catheter 110 of the present disclosure may have MRZs 96a through 96f on the outer surface of the catheter 110, residing on the inner surface of the catheter110, or both. Further, the catheter 110 may have more or fewer MRZs than is illustrated in Fig. 5, provided that the Foley catheter 110 must have at least two MRZs while any larger number of MRZs is within the scope of the present disclosure.

### Coating Tape

MRZs may be formed on surfaces of devices in a variety of manners. According to certain embodiments, MRZs may be added to devices, such as catheters, sleeves, and others, from rolls of tape, such as polymer tape, with or without modulated surfaces, each of which may be coated with an elutable or non-elutable coating. The coating tape may be selectively adhered in one or more locations of surfaces of devices forming any number of MRZs .

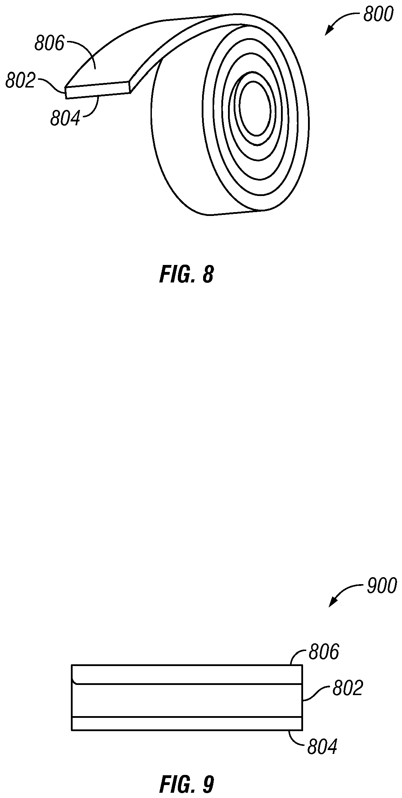

Referring to Figs. 8 and 9, in conjunction, a roll of an MRZ tape 800 includes a substrate 802, such as a polymer strip or other strip. The substrate 802 includes or is underlain with a tacker 806, such as an adhesive or other adhering agent. The tacker 804 adheres the tape 800 to a surface of a device, for example, a circumference of a sleeve, catheter or another surface of a device. The substrate 802 may include a surface structure 806, for example, any of an AET, or maybe or provide any of a detection agent, dispersers, surface modulation, Sharklet™ or Black Silicone™ surface, or other coating or structural agent.

The tape 800 is, for example, silicone or other polymer(s) of rolled strips of a variety of widths. A side of the tape 800 adheres to an underlying polymer or metallic surface of a device, such as that of a catheter, TBC, or other device, and the outward coated side of the tape is an elutable or non-elutable coating, or other coating or structure. The adherent side of the tape 800 when rolled may be masked with paper, plastic or similar material, to another material, to protect the coated side.

One possible component of the tape is polyvinylpyrillodone [PVP]. PVP can be added to silicone surfaces. The PVP may be a few microns thick, and this allows the tape to be rolled and to contain an adhesive opposing the PVP in the tape. When exposed to water containing dissolved antimicrobial drugs, PVP absorbs significant water and drugs and swells to 3-5X its original thickness. When used as a coating for tape, PVP and its dissolved drugs elute, into adjacent fluids over time.

Multiple MRZs may be possible with such tape, with respective ones of the MRZs comprising different active compositions. For example, a PVP roll may be 1cm in width and 1/1000" [1ml] thick. With such tape, a surgeon can cut short strips from the roll. A first strip may be, for example, soaked in a particular antibiotic Solution. The strip may be contacted to adhere to an outer circumference of a drainage tube or the like, forming a 1cm band as an MRZ. A second or further subsequent strip of the PVP roll may, for example, be cut and soaked in water containing a biofilm dispersive agent. This other strip may be adhered to the drainage tube along its length adjacent to the first (or other) strip, in each case to form a respective MRZ.

Additional PVP rolls may, for example, be in the form of a silicone sheet having a non-leachable electrostatic coating applied to the silicone, for example, the previously mentioned PAA/PAH coating. Such a coating firmly bonds to the underlying silicone substrate and PVP may be added atop the coating for serving as an MRZ. This PVP roll will therefore have two coatings, a bottom permanent non-eluting coating of PAA/PAH and an overlying top elutable coating of the PVP.

In other embodiments, a silicone elastomer may contain antimicrobial molecules [gentamicin, a powerful antibiotic] integrated into the silicone. The antibiotic elutes directly from the silicone over time. Such silicone elastomer may be formed as tapes or sheets to provide and serve as MRZs.

In other embodiments, a loadable tape may be provided. The loadable tape allows a surgeon to select coatings for the tape. For example, a loadable tape may be loaded in the operating room by the implant surgeon. Various coating options may be presented, for a variety of patterns and zones, to serve as MRZs.

For example, molecules can be attached to a silicone tape using the PAA/PAH coating and the tape added to a catheter or portion of the TBC. Different tapes have different molecules coated thereon. Therefore, by using the different tapes on a single catheter or TBC, multiple molecules can be placed onto the catheter. Further, the discrete region presenting a given molecule is dictated by the location and width of the tape presenting that molecule. The same principle of the tapes can be applied to sheets for application as MRZs. Such sheets and tapes can then be selected and applied to catheters or other devices by a surgeon or other health care provider. The use of tapes or sheets provide flexibility in the arrangement of AETs because the tapes can be selected and arranged according to the patient's particular circumstances, such as incision site, length of anticipated catheterization, the patient's particular bacterial (or fungal) colonization profile, particular bacterial challenges experienced at the medical facility, or any number of other factors.

### Internal Filter

According to certain embodiments, a micropore filter is fitted to a drainage tube, such as a catheter, to prevent retrograde flow of microbes in the drainage tube. Most bacteria are 1-2 microns in diameter, and submicron (or micropore) filters can filter out the bacteria. In the embodiments, catheters, such as a urinary drainage catheter, may include such a submicron filter in the drain line to block reflux access of external bag urine back into a patient's bladder.

It will be appreciated that the embodiments will be beneficial to prevent or reduce infection of the urinary tract by microbes originating from the skin of the catheterized patient. Particularly, the disclosed embodiments and the invention as claimed will reduce or eliminate the colonization of catheters by skin born bacteria or other microorganisms, will reduce or eliminate the formation of biofilms by infecting bacteria, and reduce, slow, or eliminate the migration of such biofilms from the catheter surface near the skin to bladders. A microbe resistance zone of the present disclosure may be of any size or shape and does not necessarily circumvent the entire perimeter of a catheter on which the microbe resistance zone is placed. Further, rolls of tape or sheets may form microbial resistance zones and may be applied to existing devices as retrofit. Micron filters, moreover, may reduce back flow of urine from urinary bags into the bladder.

In the foregoing specification, the invention has been described with reference to specific embodiments. However, one of ordinary skill in the art appreciates that various modifications and changes can be made without departing from the scope of the present invention as set forth in the claims below. Accordingly, the specification and figures are to be regarded in an illustrative rather than a restrictive sense, and all such modifications are intended to be included within the scope of the present invention.

Benefits, other advantages, and solutions to problems have been described above with regard to specific embodiments. However, the benefits, advantages, solutions to problems and device(s), connection(s) and element(s) that may cause any benefit, advantage, or solution to occur or become more pronounced are not to be construed as a critical, required, or essential feature or element of any or all the claims. As used herein, the terms "comprises, "comprising," or any other variation thereof, are intended to cover a non-exclusive inclusion, such that a process, method, article, or apparatus that comprises a list of elements does not include only those elements but may include other elements not expressly listed or inherent to such process, method, article, or apparatus.

## Claims

1. A cystostomy device (10) for implant in a patient, comprising:
a transcutaneous sleeve (24) with an outer surface (53) and an inner surface (55);
a catheter (26, 110) defining a passage for the flow of fluids from within the patient to outside the patient, the inner surface (55) of the transcutaneous sleeve (24) defining a path through which the catheter (26, 110) passes;
**characterized in that** the cystostomy device comprises:
an anchor (22) adapted for implantation into the biological tissue of the patient, the anchor (22) including a locking mechanism (42) configured to selectively lockingly connect to the catheter (26, 110).

2. The cystostomy device (10) of claim 1, wherein the anchor (22) comprises a material suitable for bonding with biological tissue.

3. The cystostomy device (10) of claim 1, wherein the anchor (22) comprises a material adapted for bonding with biological tissue when placed between the bladder muscle and the rectus muscle.

4. The cystostomy device (10) of claim 1, wherein the anchor (22) comprises a metallic, or solid polymeric structure to which is integrated or bonded a porous surface such as polytetrafluoroethylene, polyester or other polymeric or metallic material.

5. The cystostomy device (10) of claim 1, further comprising at least one microbe resistance zone (90a-90g, 92a-92k, 93, 94a-94h, 96a-96f).

6. The cystostomy device of claim 5, comprising a flange (58) connected to the transcutaneous sleeve (24), the flange (58) comprising at least one of the microbe resistance zones (93).

7. The cystostomy device (10) of claim 5, wherein the transcutaneous sleeve (24) comprises at least one of the microbe resistance zones (90a-90g, 92a-92k, 93).

8. The cystostomy device (10) of claim 5, wherein the catheter (26, 110) comprises at least one of the microbe resistance zones (94a-94h, 96a-96f).

9. The cystostomy device (10) of claim 1, wherein the catheter (26, 110) is removably connectable to said anchor (22).

10. The cystostomy device (10) of claim 1, comprising a flange (58) connected to the transcutaneous sleeve (24), the flange (58) being configured to reduce the migration of bacteria on a surface of the transcutaneous sleeve (24).

11. A cystostomy device (10), comprising:
a transcutaneous sleeve (24), with an outer surface (53) and an inner surface (55), the outer surface (53) lying adjacent to skin, fat, muscle or other biological tissue, and the inner surface (55) defining a path (52); and
a catheter (26, 110) defining a passage for the flow of fluids from the bladder to a surface of the patient's skin, the path (52) of the inner surface (55) of the transcutaneous sleeve (24) allows passage of the catheter (26, 110);
**characterized in that** the cystostomy device comprises:
an anchor (22) adapted for implantation into biological tissue that removably connects to the catheter (26, 110);
at least one microbe resistance zone (90a-90g, 92a-92k, 93, 94a-94h, 96a-96f) of either of the transcutaneous sleeve (24) and the catheter (26, 110);
wherein the transcutaneous sleeve (24) is removably connected to the catheter (26, 110).

12. The cystostomy device (10) of claim 11, wherein the at least one microbe resistance zone (90a-90g, 92a-92k, 93, 94a-94h, 96a-96f) comprises a first microbe resistance zone containing a first antimicrobial eradication therapy and a second microbe resistance zone containing a second antimicrobial eradication therapy distinct from the first antimicrobial eradication therapy.

13. The cystostomy device (10) of claim 12,
wherein the first microbe resistance zone includes a surface modulation; and
wherein the second microbe resistance zone is selected from the group of: antibiotic, antifungal, antiseptic, biofilm inhibitor, dispersing agent, a detector, an enzyme-linked immunosorbent assay, elutable coating, non-elutable coating, and combinations.

14. The cystostomy device (10) of claim 11, wherein the at least one microbe resistance zone (90a-90g, 92a-92k, 93, 94a-94h, 96a-96f) is selected from the group of:
surface modulations, antibiotic, antifungal, antiseptic, biofilm inhibitor, dispersing agent, a detector, an enzyme-linked immunosorbent assay, elutable coating, non-elutable coating, and combinations.

15. The cystostomy device (10) of claim 11, wherein the catheter (26, 110) comprises at least one of the microbe resistance zone (94a-94h, 96a-96f).

16. The cystostomy device (10) of claim 11, comprising a flange (58) configured to reduce contamination of the outer surface (53) of said transcutaneous sleeve (24), wherein the flange (58) comprises at least one of the microbe resistance zone (90a-90g, 92a-92k, 93).

## Patentansprüche

1. Zystostomievorrichtung (10) zur Implantation in einen Patienten, die Folgendes umfasst:
eine transkutane Hülse (24) mit einer Außenfläche (53) und einer Innenfläche (55);
einen Katheter (26, 110), der einen Durchgang für den Fluss von Fluiden von innerhalb des Patienten zur Außenseite des Patienten definiert, wobei die Innenfläche (55) der transkutanen Hülse (24) einen Pfad definiert, durch den der Katheter (26, 110) hindurchtritt;
**dadurch gekennzeichnet, dass** die Zystostomievorrichtung Folgendes umfasst:
einen Anker (22), der zur Implantation in das biologische Gewebe des Patienten angepasst ist, wobei der Anker (22) einen Verriegelungsmechanismus (42) umfasst, der konfiguriert ist, sich selektiv verriegelnd mit dem Katheter (26, 110) zu verbinden.

2. Zystostomievorrichtung (10) nach Anspruch 1, wobei der Anker (22) ein Material umfasst, das zur Verbindung mit dem biologischen Gewebe geeignet ist.

3. Zystostomievorrichtung (10) nach Anspruch 1, wobei der Anker (22) ein Material umfasst, das zur haftschlüssigen Verbindung mit dem biologischen Gewebe angepasst ist, wenn er zwischen dem Blasenmuskel und dem Rektusmuskel platziert wird.

4. Zystostomievorrichtung (10) nach Anspruch 1, wobei der Anker (22) eine Metall- oder feste Polymerstruktur umfasst, in die/an der eine poröse Oberfläche wie z. B. Polytetrafluorethylen, Polyester oder anderes Polymer- oder Metallmaterial integriert oder befestigt ist.

5. Zystostomievorrichtung (10) nach Anspruch 1, die weiters zumindest eine Mikrobenbeständigkeitszone (90a-90g, 92a-92k, 94a-94h, 96a-96f) umfasst.

6. Zystostomievorrichtung nach Anspruch 5, die einen Flansch (58) umfasst, der mit der transkutanen Hülse (24) verbunden ist, wobei der Flansch (58) zumindest eine der Mikrobenbeständigkeitszonen (93) umfasst.

7. Zystostomievorrichtung (10) nach Anspruch 5, wobei die transkutane Hülse (24) zumindest eine der Mikrobenbeständigkeitszonen (90a-90g, 92a-92k, 93) umfasst.

8. Zystostomievorrichtung (10) nach Anspruch 5, wobei der Katheter (26, 110) zumindest eine der Mikrobenbeständigkeitszonen (94a-94h, 96a-96f) umfasst.

9. Zystostomievorrichtung (10) nach Anspruch 1, wobei der Katheter (26, 110) trennbar mit dem Anker (22) verbindbar ist.

10. Zystostomievorrichtung (10) nach Anspruch 1, die einen Flansch (58) umfasst, der mit der transkutanen Hülse (24) verbunden ist, wobei der Flansch (58) konfiguriert ist, um die Migration von Bakterien auf einer Oberfläche der transkutanen Hülse (24) zu reduzieren.

11. Zystostomievorrichtung (10), die Folgendes umfasst:
eine transkutane Hülse (24) mit einer Außenfläche (53) und einer Innenfläche (55), wobei die Außenfläche (53) an Haut, Fett, einem Muskel oder einem anderen biologischen Gewebe anliegt und die Innenfläche (55) einen Pfad (52) definiert; und
einen Katheter (26, 110), der einen Durchgang für den Strom von Fluiden von der Blase zu einer Hautoberfläche des Patienten definiert, wobei der Pfad (52) der Innenfläche (55) der transkutanen Hülse (24) das Hindurchtreten des Katheters (26, 110) ermöglicht;
**dadurch gekennzeichnet, dass** die Zystostomievorrichtung Folgendes umfasst:
einen Anker (22), der zur Implantation in biologisches Gewebe angepasst ist und trennbar mit dem Katheter (26, 110) verbunden ist;
zumindest eine Mikrobenbeständigkeitszone (90a-90g, 92a-92k, 94a-94h, 96a-96f) von einem aus der transkutanen Hülse (24) und dem Katheter (26, 110);
wobei die transkutane Hülse (24) trennbar mit dem Katheter (26, 110) verbunden ist.

12. Zystostomievorrichtung (10) nach Anspruch 11, wobei die zumindest eine Mikrobenbeständigkeitszone (90a-90g, 92a-92k, 94a-94h, 96a-96f) eine erste Mikrobenbeständigkeitszone, die eine erste antimikrobielle Eradikationstherapie enthält, und eine zweite Mikrobenbeständigkeitszone, die eine zweite antimikrobielle Eradikationstherapie enthält, die sich von der ersten antimikrobiellen Eradikationstherapie unterscheidet, umfasst.

13. Zystostomievorrichtung (10) nach Anspruch 12,
wobei die erste Mikrobenbeständigkeitszone eine Oberflächenmodulation umfasst, und
wobei die zweite Mikrobenbeständigkeitszone aus folgender Gruppe ausgewählt ist: Antibiotika, Antimykotika, Antiseptika, Biofilminhibitoren, Dispergiermittel, Detektor, enzymgekoppelter Immunsorptionstest, eluierbare Beschichtung, nicht eluierbare Beschichtung und Kombinationen.

14. Zystostomievorrichtung (10) nach Anspruch 11, wobei die zumindest eine Mikrobenbeständigkeitszone (90a-90g, 92a-92k, 94a-94h, 96a-96f) aus folgender Gruppe ausgewählt ist: Oberflächenmodulationen, Antibiotika, Antimykotika, Antiseptika, Biofilminhibitor, Dispergiermittel, Detektor, enzymgekoppelter Immunsorptionstest, eluierbare Beschichtung, nicht eluierbare Beschichtung und Kombinationen.

15. Zystostomievorrichtung (10) nach Anspruch 11, wobei der Katheter (26, 110), zumindest eine der Mikrobenbeständigkeitszonen (94a,94h, 96a-96f) umfasst.

16. Zystostomievorrichtung (10) nach Anspruch 11, die einen Flansch (58) umfasst, der konfiguriert ist, um die Kontamination der Außenfläche (53) der transkutanen Hülse 824) zu verringern, wobei der Flansch (58) zumindest eine der Mikrobenbeständigkeitszonen (90a-90g, 92a-92k, 93) umfasst.

## Revendications

1. Dispositif de cystostomie (10) destiné à être implanté sur un patient, comprenant :
un manchon transcutané (24) avec une surface externe (53) et une surface interne (55) ;
un cathéter (26, 110) définissant un passage pour l'écoulement des fluides de l'intérieur du patient à l'extérieur du patient, la surface interne (55) du manchon transcutané (24) définissant une trajectoire à travers laquelle passe le cathéter (26, 110) ;
**caractérisé en ce que** le dispositif de cystostomie comprend :
un ancrage (22) adapté pour l'implantation dans le tissu biologique du patient, l'ancrage (22) comprenant un mécanisme de blocage (42) configuré pour se raccorder sélectivement par blocage au cathéter (26, 110).

2. Dispositif de cystostomie (10) selon la revendication 1, dans lequel l'ancrage (22) comprend un matériau approprié pour la liaison avec le tissu biologique.

3. Dispositif de cystostomie (10) selon la revendication 1, dans lequel l'ancrage (22) comprend un matériau adapté pour se lier avec le tissu biologique lorsqu'il est placé entre le muscle de la vessie et le muscle droit.

4. Dispositif de cystostomie (10) selon la revendication 1, dans lequel l'ancrage (22) comprend une structure métallique ou polymère solide à laquelle est intégrée ou reliée une surface poreuse telle qu'un matériau en polytétrafluoroéthylène, polyester ou un autre matériau polymère ou métallique.

5. Dispositif de cystostomie (10) selon la revendication 1, comprenant en outre au moins une zone de résistance microbienne (90a-90g, 92a-92k, 93, 94a-94h, 96a-96f) .

6. Dispositif de cystostomie (10) selon la revendication 5, comprenant une bride (58) raccordée au manchon transcutané (24), la bride (58) comprenant au moins l'une des zones de résistance microbienne (93).

7. Dispositif de cystostomie (10) selon la revendication 5, dans lequel le manchon transcutané (24) comprend au moins l'une des zones de résistance microbienne (90a-90g, 92a-92k, 93).

8. Dispositif de cystostomie (10) selon la revendication 5, dans lequel le cathéter (26, 110) comprend au moins l'une des zones de résistance microbienne (94a-94, 96a-96f) .

9. Dispositif de cystostomie (10) selon la revendication 1, dans lequel le cathéter (26, 110) peut être raccordé, de manière amovible, audit ancrage (22).

10. Dispositif de cystostomie (10) selon la revendication 1, comprenant une bride (58) raccordée au manchon transcutané (24), la bride (58) étant configurée pour réduire la migration des bactéries sur une surface du manchon transcutané (24).

11. Dispositif de cystostomie (10) comprenant :
un manchon transcutané (24) avec une surface externe (53) et une surface interne (55), la surface externe (53) étant adjacente à la peau, à la graisse, à un muscle ou à un autre tissu biologique et la surface interne (55) définissant une trajectoire (52) ; et
un cathéter (26, 110) définissant un passage pour l'écoulement des fluides de la vessie à une surface de la peau du patient, la trajectoire (52) de la surface interne (55) du manchon transcutané (24) permet le passage du cathéter (26, 110) ;
**caractérisé en ce que** le dispositif de cystostomie comprend :
un ancrage (22) adapté pour l'implantation dans le tissu biologique qui se raccorde, de manière amovible, au cathéter (26, 110) ;
au moins une zone de résistance microbienne (90a-90g, 92a-92k, 93, 94a-94h, 96a-96f) de chacun parmi le manchon transcutané (24) et le cathéter (26, 110) ;
dans lequel le manchon transcutané (24) est raccordé, de manière amovible, au cathéter (26, 110).

12. Dispositif de cystostomie (10) selon la revendication 11, dans lequel la au moins une zone de résistance microbienne (90a-90g, 92a-92k, 93, 94a-94h, 96a-96f) comprend une première zone de résistance microbienne contenant une première thérapie d'éradication antimicrobienne et une seconde zone de résistance microbienne contenant une seconde thérapie d'éradication antimicrobienne distincte de la première thérapie d'éradication antimicrobienne.

13. Dispositif de cystostomie (10) selon la revendication 12,
dans lequel la première zone de résistance microbienne comprend une modulation de surface ; et
dans lequel la seconde zone de résistance microbienne est sélectionnée dans le groupe comprenant : un antibiotique, un antifongique, un antiseptique, un inhibiteur de film biologique, un agent de dispersion, un détecteur, un dosage immunoenzymatique, un revêtement éluable, un revêtement non éluable et leurs combinaisons.

14. Dispositif de cystostomie (10) selon la revendication 11, dans lequel la au moins une zone de résistance microbienne (90a-90g, 92a-92k, 93, 94a-94h, 96a-96f) est sélectionnée dans le groupe comprenant : des modulations de surface, un antibiotique, un antifongique, un antiseptique, un inhibiteur de film biologique, un agent de dispersion, un détecteur, un dosage immunoenzymatique, un revêtement éluable, un revêtement non éluable et leurs combinaisons.

15. Dispositif de cystostomie (10) selon la revendication 11, dans lequel le cathéter (26, 110) comprend au moins l'une des zones de résistance microbienne (94a-94h, 96a-96f) .

16. Dispositif de cystostomie (10) selon la revendication 11, comprenant une bride (58) configurée pour réduire la contamination de la surface externe (53) dudit manchon transcutané (24), dans lequel la bride (58) comprend au moins l'une des zones de résistance microbienne (90a-90g, 92a-92k, 93) .
